# EUROPÄISCHE PATENTANMELDUNG

(11) **EP 0 578 163 A1**
(43) Veröffentlichungstag der Anmeldung: **12.01.1994**
(21) Anmeldenummer: 93110705.6
(22) Anmeldetag: 05.07.1993
(51) Int. Cl.: C07D 217/26

(54) **Verfahren zur Herstellung von D- bzw.L-bzw. D,L-1,2,3,4-Tetrahydroisochinolin-3-Carbonsäure**

(30) Priorität: 09.07.1992 DE 4222549; 05.09.1992 DE 4229758
(71) Anmelder: HOECHST AKTIENGESELLSCHAFT, D-65926 Frankfurt am Main (DE)
(72) Erfinder: Martin, Wolfgang, Dr., D-65779 Kelkheim/Ts. (DE)

(57) **Zusammenfassung**

Beschrieben wird ein Verfahren zur Herstellung von D- bzw. L- bzw. D,L-1,2,3,4-Tetrahydroisochinolin-3-carbonsäure. Es besteht darin, daß man Phenylalaninalkylester mit Formaldehyd in Trifluoressigsäure zum entsprechenden 1,2,3,4-Tetrahydroisochinolin-3-carbonsäureester zyklisiert und diesen anschließend zur entsprechenden 1,2,3,4-Tetrahydroisochinolin-3-carbonsäure verseift.

## Beschreibung

D-1,2,3,4-Tetrahydroisochinolin-3-carbonsäure, eine in der Natur nicht vorkommende Iminosäure, ist ein wichtiges Synthon für Peptidsynthesen, so auch für Arzneimittelsynthesen.

Es ist bekannt, daß 1,2,3,4-Tetrahydroisochinolin-3-carbonsäure durch Pictet-Spengler Reaktion mit 56 % Ausbeute (D,L-Form; Ber. dtsch. Chem. Ges. 44, 1911, 2030) bzw. 68 % Ausbeute (L-Form, JP 02 193 969, referiert in CA 1991, 114, 61951e) hergestellt werden kann.
Die Reaktionsbedingungen dieser bekannten Pictet-Spengler-Reaktion (Erhitzen einer Aminosäure mit einem Aldehyd in konzentrierter Salzsäure) sind so drastisch, daß teilweise Racemisierung nicht vermieden werden kann.

In JP 02 293 969 ist z.B. für die Reaktion von Phenylalanin mit Paraformaldehyd ein Enantiomeren-Überschuß (ee) von 74,1 %, für die Reaktion von Phenylalanin mit Formaldehyd nur 66,6 % ee angegeben, es findet also ca. 13 % bzw. ca. 17 % Racemisierung statt. Die Ausbeuten liegen bei 68 % bzw. 56 %. Dies wurde durch unsere eigenen Untersuchungen bestätigt; hier wurde bei Standard-Pictet-Spengler-Reaktionen ein Racemisierungsgrad von mindestens 10 % gefunden. Außerdem sinkt durch die teilweise Racemisierung und die deshalb zur Herstellung optisch reiner Produkte erforderliche(n) Umkristallisation(en) die Ausbeute leicht auf unter 30 %.

Dagegen verläuft die erfindungsgemäße Reaktion - ausgehend von D-Phenylalaninmethylester anstatt der freien Säure - sehr schonend und glatt schon bei Raumtemperatur. Besonders hervorzuheben ist der völlige Erhalt der optischen Reinheit (z.B. 99,9 % ee im Falle von D-Tic) und die sehr hohe chemische Reinheit (> 99 %), mit der das Produkt der zweistufigen Reaktion ohne zusätzlichen Reinigungsaufwand anfällt. Hierdurch werden auch sehr hohe Ausbeuten (80 - 84 %) erzielt.

Ein weiterer enormer Vorteil des erfindungsgemäßen Verfahrens besteht in der Vermeidung der Bildung des carcinogenen Bischlormethylethers (gravierender Nachteil der üblichen Pictet-Spengler-Reaktion).

Aus der EP 421 436 ist der Ringschluß von Derivaten des Phenylalanins zu 1,2,3,4-Tetrahydroisochinolin-3-carbonsäure-Derivaten mit Hilfe von Aldehyden und Säuren bekannt, jedoch handelt es sich dort bei den Phenylalaninderivaten stets um die Zyklisierung von Urethanen, nicht aber von Verbindungen mit freier NH₂-Gruppe, abgesehen davon, daß nach EP 421 436 stets noch Substituenten im aromatischen Ring enthalten sind.

Das erfindungsgemäße Verfahren besteht darin, daß D-1,2,3,4-Tetrahydroisochinolin-3-carbonsäure aus D-Phenylalanin in optisch reiner Form in einer 3-stufigen Synthese einfach und kostengünstig dargestellt werden kann: 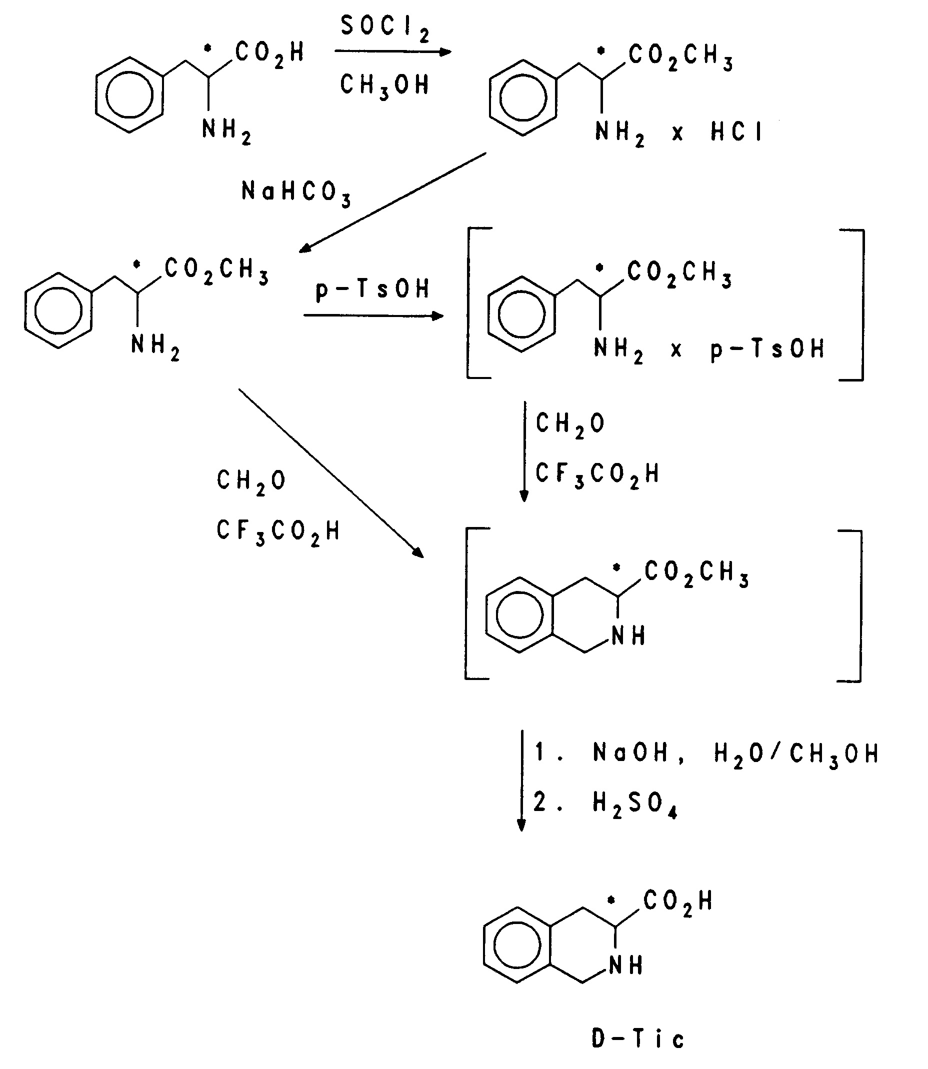
Dabei wird D-Phenylalanin z. B. mit Thionylchlorid/Methanol in den D-Phenylalaninmethylester (hydrochlorid) überfuhrt (Ausbeute: 97 - 99 % d. Th.).

In dem Molekül noch enthaltene, salzartig gebundene Salzsäure entfernt man vollständig durch Neutralisation, beispielsweise mit Natriumbicarbonat, gefolgt von Extraktion des freien Esters mit Ethylacetat oder Methylenchlorid und Waschen mit Wasser. Man kann die freie D-Phenylalaninesterbase direkt mit Formaldehyd bzw. Paraformaldehyd in Trifluoressigsäure zu D-Tic zyklisieren. Aus Stabilitätsgründen, d. h. um D-1,2,3,4-Tetrahydroisochinolin-3-carbonsäure in besonders hoher Ausbeute und guter Qualität zu erhalten, kann man alternativ die vorher in Lösung über wasserfreiem Natriumsulfat getrocknete Base als Salz, z. B. als p-Toluolsulfonsäuresalz bzw. als Trichloracetat bzw. als Methansulfonat bzw. als Sulfat bzw. als Amidosulfonat, mit Paraformaldehyd in Trifluoressigsäure umsetzen. Die Zyklisierungsreaktion zu D-1,2,3,4-Tetrahydroisochinolin-3-carbonsäuremethylester erfolgt schon bei Raumtemperatur während 10 - 20 Std. zu ca. 80 %, besser noch (praktisch quantitativ) beim Erhitzen auf 80°C bis Rückflußtemperatur während 6 - 7 Stunden. Der Reaktionsfortschritt bzw. die Vollständigkeit der Reaktion ist dünnschichtchromatographisch leicht zu prüfen [Kieselgel, Merck_{F254}; Chloroform/Essigsäure/Methanol/Wasser = 60/3/45/14 (VV); D-Phenylalanin: Rf = 0,50 (violett, mit Ninhydrin und Erwärmen auf ca. 150°C); D-1,2,3,4-Tetrahydroisochinolin-3-carbonsäure-methylester: Rf = 0,85 (gelb); D-1,2,3,4-Tetrahydroisochinolin-3-carbonsäure: RF = 0,55 (gelb)].

D-1,2,3,4-Tetrahydroisochinolin-3-carbonsäure-methylester wird schließlich durch alkalische Verseifung bei Raumtemperatur während 3 Std., gefolgt von Ansäuern mit Schwefelsäure auf pH 6,5, in D-1,2,3,4-Tetrahydroisochinolin-3-carbonsäure überführt.

Die Vorteile des Verfahrens bestehen in dem eindeutigen Reaktionsverlauf auf allen Stufen, in hoher Gesamt-Ausbeute (ca. 80 % d. Th.), in hoher Produktreinheit (ca. 100 %ig, ohne zusätzliche Reinigung), in hoher optischer Reinheit (< 0,1 % L-Isomer).

Alkyl bedeutet, sofern nicht anders angegeben, (C₁-C₈)-Alkyl, vorzugsweise (C₁-C₄)-Alkyl. Besonders bevorzugt sind Methyl- oder Ethylgruppen.

Salze der D-Phenylalaninester sind solche mit anorganischen oder organischen Säuren, wie Salzsäure, Schwefelsäure, p-Toluolsulfonsäure, Trichloressigsäure, Methansulfonsäure, Amidosufonsäure und anderen.

In gleicher Weise kann auch das entsprechende L-Isomer der 1,2,3,4-Tetrahydroisochinolin-3-carbonsäure in optisch reiner Form hergestellt werden (und folglich auch das Racemat).

### Beispiele

### Beispiel 1

### D-phenylalaninmethylester-hydrochlorid (Ausgangsmaterial)

200 g D-Phenylalanin werden in 1200 ml Methanol suspendiert und bei 0 - 5°C mit 340 g Thionylchlorid tropfenweise versetzt. Man rührt 1 Stunde bei ca. 0°C, danach 3,5 Stunden bei 50°C, engt im Vakuum zu einem Kristallbrei ein und versetzt diesen mit 700 ml Diisopropylether. Man rührt die Kristallsuspension 2 Std. im Eisbad, saugt ab und wäscht mit 300 ml Diisopropylether. Trocknung bei 30°C im Vakuum.
Ausbeute: 257,4 g (99 %). Gehalt: 100 %.

### Beispiel 2

30 g D-Phenylalaninmethylester-hydrochlorid (Beispiel 1) werden in einer Lösung von 23 g Natriumbicarbonat in 220 ml Wasser gerührt und in 300 ml Methylenchlorid aufgenommen. Nach Phasentrennung wird die wäßrige Phase mit insgesamt 275 ml Methylenchlorid extrahiert, und die vereinigten Methylenchloridphasen werden 2 x mit je 100 ml Wasser chloridfrei gewaschen. Nach Trocknung der Methylenchloridphase über wasserfreiem Natriumsulfat und Einengen im Vakuum erhält man 25,0 g D-Phenylalaninmethylester-Base, die durch Zusatz von 26,4 g p-Toluolsuffonsäurehydrat in das Toluolsulfonat überführt wird. Man versetzt anschließend mit 300 ml Trifluoressigsäure und 4,69 g Paraformaldehyd, erhitzt 6,5 Stunden zum Rückfluß und destilliert anschließend Trifluoressigsäure im Vakuum ab. Man gibt zum öligen Rückstand 168 ml Wasser und 76 ml halbkonzentrierte Natronlauge ad pH 7. Sodann werden 11,16 g Ätznatron, in 470 ml Methanol gelöst, zugegeben. Die Lösung wird 3 Stunden bei Raumtemperatur gerührt und danach im Vakuum eingeengt. Die wäßrige, zurückbleibende Lösung wird mit 0,35 g Natriumdithionit und 1 g Aktivkohle 20 Minuten behandelt und filtriert. Das Filtrat wird mit Wasser auf 600 ml aufgefüllt, und daraus wird mit 15 %iger Schwefelsäure D-1,2,3,4-Tetrahydroisochinolin-3-carbonsäure ad pH 6,35 gefällt. Man rührt die Suspension 1 Stunde im Eisbad, saugt ab und wäscht mit Wasser sulfatfrei. Trocknung im Vakuum bei 30°C.
Ausbeute: 20,0 g (81 %).

Zum Vergleich: Die Zyklisierung des D-Phenylalaninmethylester-hydrochlorids verlief mit 84 % Ausbeute.

### Beispiel 3

Eine Suspension von 20 g D-Phenylalaninmethylester-hydrochlorid in 132 ml Methylenchlorid wird mit einer Lösung von 8 g Natriumbicarbonat in 82 ml Wasser versetzt und 15 Minuten gerührt. Man trennt die Phasen und extrahiert die wässrige Phase erneut mit 26 ml Methylenchlorid. Die Methylenchloridphasen werden vereinigt und mit Wasser chloridfrei gewaschen. Nach Trocknung über Natriumsulfat und Filtration wird durch Zutropfen von 7 g konz. Schwefelsäure unter Rühren das D-Phenylalaninmethylestersuffat gebildet. Die resultierende Kristallsuspension wird im Vakuum zur Trockne eingedampft. Man fügt 3,0 g Paraformaldehyd und 114 ml trifluoressigsäure zu, erhitzt zum Rückfluß (82°C) und hält die Mischung 6,5 Stunden bei dieser Temperatur. Danach wird die Trifluoressigsäure im Vakuum vollständig abdestilliert. Der Rückstand wird mit 112 ml Wasser und 56 ml halbkonz. Natronlauge ad pH 6,8 bis 7,0 versetzt, und danach mit einer Lösung von 7,0 g Ätznatron in 310 ml Methanol. Nach dreistündigem Rühren bei 25°C klärt man mit 0,7 g Aktivkohle, destilliert das Methanol im Vakuum weitgehend ab, ergänzt das Volumen mit Wasser auf 400 ml und fällt D-Tetrahydroisochinolincarbonsäure durch Zutropfen von ca. 44 ml 15 %iger Schwefelsäure ad pH 6,2 aus. Das Produkt wird abgesaugt, mit Wasser sulfatfrei gewaschen und im Vakuum bei 40°C getrocknet. Ausbeute: 12,5 g.

### Beispiel 4

100 g D-Phenylalaninmethylester-hydrochlorid (Beispiel 1) werden in einer Lösung von 40 g Natriumbicarbonat in 410 ml Wasser gerührt und in 660 ml Methylenchlorid aufgenommen. Nach Phasentrennung wird die wäßrige Phase mit insgesamt 260 ml Methylenchlorid extrahiert, und die vereinigten Methylenchloridphasen werden mit Wasser chloridfrei gewaschen. Nach Trocknung der Methylenchloridphase über wasserfreiem Natriumsulfat und Einengen im Vakuum erhält man 75,6 g D-Phenylalaninmethylester-Base.

Man versetzt anschließend mit 570 ml Trifluoressigsäure und 15 g Paraformaldehyd, erhitzt 6,5 Stunden zum Rückfluß und destilliert anschließend Trifluoressigsäure im Vakuum ab. Man gibt zum öligen Rückstand 560 ml Wasser und 270 ml 15 %ige Natronlauge. Sodann werden 35 g Ätznatron, in 1550 ml Methanol gelöst, zugegeben. Die Lösung wird 3 Stunden bei Raumtemperatur gerührt und danach im Vakuum eingeengt. Die zurückbleibende wäßrige Lösung wird mit 1,15 g Natriumdithionit und 3,3 g Aktivkohle 30 Minuten behandelt und filtriert. Das Filtrat wird mit Wasser auf 2 l aufgefüllt, und daraus wird mit 15 %iger Schwefelsäure D-1,2,3,4-Tetrahydroisochinolin-3-carbonsäure ad pH 6,5 gefällt. Man rührt die Suspension 1 Stunde im Eisbad, saugt ab und wäscht mit Wasser sulfatfrei. Trocknung im Vakuum bei 30°C.
Ausbeute: 58,9 g (72 % d. Th.)

### Beispiel 5

12,1 g L-Phenylalaninbenzylester-hydrochlorid und 1,40 g Paraformaldehyd werden in 52 ml Trifluoressigsäure 6,5 Stunden am Rückfluß gekocht. Von etwas abgeschiedenem Harz wird dekantiert. Die Trifluoressigsäurelösung wird im Vakuum zu einem Öl eingeengt, mit 20,5 ml 16 %iger Natronlauge und 50 ml Wasser auf pH 7,0 gebracht und danach mit einer Lösung von 3,33 g Ätznatron in 140 ml Wasser ad pH 9 versetzt. Man rührt 3 Stunden bei 20 bis 25°C, klärt mit 0,18 g Natriumdithionit und 0,5 g Aktivkohle und destilliert das Methanol im Vakuum ab. Man fügt Wasser bis auf 250 ml zu und fällt L-Tetrahydroisochinolin-3-carbonsäure mit 15 %iger Schwefelsäure bei pH 6,35 aus. Man wäscht das Produkt mit 220 ml Wasser sulfatfrei und trocknet es im Vakuum.
Ausbeute: 4,02 g (55 % d. Th.).

## Patentansprüche

1. Verfahren zur Herstellung von D-1,2,3,4-Tetrahydroisochinolin-3-carbonsäure, dadurch gekennzeichnet, daß man D-Phenylalaninalkyl- oder Aralkylester mit Formaldehyd in Trifluoressigsäure zum entsprechenden D-1,2,3,4-Tetrahydroisochinolin-3-carbonsäure-Ester zyklisiert und diesen anschließend zu D-1,2,3,4-Tetrahydroisochinolin-3-carbonsäure verseift.

2. Verfahren nach Anspruch 1, dadurch gekennzeichnet, daß man den D-Phenylalaninmethylester einsetzt.

3. Verfahren nach Anspruch 1, dadurch gekennzeichnet, daß man ein entsprechendes Salz der D-Phenylalaninester einsetzt.

4. Verfahren zur Herstellung von L-1,2,3,4-Tetrahydroisochinolin-3-carbonsäure, dadurch gekennzeichnet, daß man L-Phenylalaninester oder ein entsprechendes Salz der L-Phenylalaninester einsetzt.

5. Verfahren zur Herstellung von D,L-1,2,3,4-Tetrahydroisochinolin-3-carbonsäure, dadurch gekennzeichnet, daß man D,L-Phenylalaninester oder ein entsprechendes Salz der racemischen D,L-Phenylalanin-alkyl- oder - aralkyl-ester einsetzt.
